Europäisches Patentamt

European Patent Office

Office européen des brevets

(11) Publication number : **0 519 727 A1**

# (12) EUROPEAN PATENT APPLICATION

(21) Application number : **92305625.3**

(22) Date of filing : **19.06.92**

(51) Int. Cl.$^5$ : **A61K 7/48, A61K 7/06, A61K 7/043**

(30) Priority : **21.06.91 GB 9113484**

(43) Date of publication of application :
**23.12.92 Bulletin 92/52**

(84) Designated Contracting States :
**AT BE CH DE DK ES FR GB GR IT LI NL PT SE**

(71) Applicant : **UNILEVER PLC**
**Unilever House Blackfriars**
**London EC4P 4BQ (GB)**
(84) **GB**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Hewitt, Jacqueline Diane**
**6a Barsbank Lane**
**Lymm, Cheshire (GB)**
Inventor : **Rosser, David Arthur**
**The Sycamores, The Mounts**
**Heswall, Wirral (GB)**
Inventor : **Naidoo, Ananda**
**165 Spencer Road**
**Clare Estate, 4091 Durban (ZA)**

(74) Representative : **Ford, Michael Frederick et al**
**MEWBURN ELLIS 2 Cursitor Street**
**London EC4A 1BQ (GB)**

(54) **Cosmetic composition.**

(57) A cosmetic oleogel suitable for topical application to the human body suface comprises :
   i) from 5 to 95% by weight of a cosmetically acceptable solvent chosen from polyol fatty acid polyesters, and mixtures thereof, the polyol having at least 3 free hydroxyl groups, at least 60% of which are then esterified with one or more fatty acids having from 8 to 22 carbon atoms ;
provided that when the polyol fatty acid polyester is derived from a polyol having 3 free hydroxyl groups, then at least one fatty acid with which it is esterified is chosen from alkanoic or alkenoic fatty acids having from 13 to 33 carbon atoms ; the polyol fatty acid polyester having an Iodine Value of <180 ; and
   ii) from 5 to 70% by weight of a cosmetically acceptable hydrophobic polymer thickener substantially free from cross-l ;inking which is soluble in the solvent.

EP 0 519 727 A1

FIELD OF THE INVENTION

The invention relates to a cosmetic composition, particularly an oleogel which can be applied topically to the human body surface, especially the skin (including the mucosae), the hair and the nails. The cosmetic oleogel can be used as such, for example to provide an occlusive layer wherever it is applied, or it can be employed as a carrier or vehicle for ingredients intended to impart to the region of application some cosmetic or pharmaceutical benefit.

BACKGROUND AND PRIOR ART

Hydrocarbons, such as petrolatum (also known as petroleum jelly or soft paraffin) have been used for many years for topical application to human skin for providing an occlusive film thereon to prevent water loss to the environment, thereby allowing water diffusing from the underlying tissues to accumulate in the stratum corneum. Petrolatum has also been used as an ingredient of skin care products, such as hand creams and lotions, and has also featured in hair grooming or conditioning products, particularly as a setting aid to maintain hair in a desired configuration.

It is, however, widely recognised that petrolatum possesses a relatively narrow spectrum of sensory or aesthetic properties. In particular, it can impart to the skin and hair an uncomfortable feeling of warmth, in addition to a sticky, waxy feel, and this has restricted its use to barrier products such as petrolatum itself and hand creams containing it, where a temporary functional protective film on the skin is desired, and to hair dressings such as pomades.

It is also recognised that petrolatum is derived from fossil fuels, whose supply is non-renewable.

In view of disadvantages such as those attributable to traditional petrolatum, there exists a need to locate an alternative occlusive product that has all the desirable attributes of emolliency and occlusivity of petrolatum, without serious negative subjective properties. Also, such an alternative product ideally should contain a major proportion of plant-derived material to suit environmental, ecological and personal health care needs.

The problems of preparing an ointment base in the form of a gel with controlled consistency characteristics has been reviewed in GB 1 370 699 (Dynamit Nobel Aktiengesellschaft). Here it is stated that coconut oil containing longer-chained fatty acid and lauric acid is not suitable for use as an ointment base due to the strong dependence of the consistency on temperature. It is also proposed to mix short chain fatty acids with long chain fatty acids and esterify the mixture with glycerine to obtain an ointment base, like soft paraffin with good spreadability, but with unsatisfactory consistency. A further proposal involves bringing liquid paraffin into an ointment-like consistency by working with polyethylene, but this is seen as unsatisfactory as the use of hydrocarbons in pharmaceuticals and cosmetics is limited, due to the hydrophobic properties of these products. Yet a further proposal concerns the attempt to give liquid triglycerides, such as almond oil, an ointment-like consistency by means of additives, such as natural waxes, for example spermaceti and beeswax. However, even this proposal is said not to fulfil the demands made on ointment bases due to their lack of stability.

Against this background, Dynamit conclude that it has hitherto proved impossible to convert normally liquid, saturated fatty acid esters into a spreadable ointment base. Their solution to the problem of developing an ointment base which remains spreadable over a wide temperature range of at least 0 to 40°C and which is not prone to rancidity, is to employ glycerine esters of saturated fatty acids with 8 to 12 carbon atoms in the molecule. One aspect of this approach is to form an ointment base from an intimate mixture of a saturated triglyceride wherein the fatty acid residues each contain 8 to 12 carbon atoms, and polyethylene having an average molecular weight of 1,000 to 20,000 or an ethylene/vinyl acetate copolymer, as a gelling agent. The triglyceride starting material is further characterised as having an acid number of <1, a saponification number of 220-345 and an iodine number (ie Iodine Value) of <1.

While appreciating the shortcomings of the prior proposals reviewed by Dynamit, we have found that fully saturated short chain fatty acids with a very low Iodine Value are unreasonably costly to employ and in any case do not yield a sufficiently attractive ointment base to meet the demands of the cosmetics industry today.

We have, however, now discovered in contrast to the teaching of Dynamit that certain vegetable oils, that are suitable for cosmetic use, can be treated with a special polymer in a controlled manner, to increase the viscosity of the oil, thereby to provide an oleogel having predictable rheological and melting characteristics. This oleogel can, moreover, be engineered to possess the appearance and desirable physical properties of petrolatum, in particular optical properties, temperature stability, occlusivity and emolliency, without some of the undesirable characteristics of this product referred to earlier.

The use of indigenous vegetable oils for this purpose is also of particular value in those territories, for examples parts of Africa and the Indian subcontinent, where hydrocarbon oils which could be converted to oleogels are a scarce and expensive commodity.

It should be explained that by "oleogels", is meant oleagenous (ie oily) materials which have been thickened to provide gel-like properties.

We have also discovered that selected polyol fatty acid polyesters other than vegetable oils, such as those derived from natural sugars and vegetable oils, can be treated in a similar manner with the special polymer to provide an oleogel which also has the appearance and physical characteristics of petrolatum, without the undesirable sensory properties previously alluded to.

We have additionally discovered that mixtures of these vegetable oils and polyol fatty acid polyesters can be treated with the special polymer to provide products which also have the desirable physical properties of petrolatum.

## DEFINITION OF THE INVENTION

According to the invention, there is provided a cosmetic oleogel suitable for topical application to the human body surface, which comprises:

(i) from 5 to 95% by weight of a cosmetically acceptable solvent chosen from polyol fatty acid polyesters, and mixtures thereof, the polyol having at least 3 free hydroxyl groups, at least 60% of which are then esterified with one or more fatty acids having from 8 to 22 carbon atoms;

provided that when the polyol fatty acid polyester is derived from a polyol having 3 free hydroxyl groups, then at least one fatty acid with which it is esterified is chosen from alkanoic or alkenoic fatty acids having from 13 to 22 carbon atoms, the polyol fatty acid polyester having an Iodine Value of <180;

(ii) from 5 to 70% by weight of a cosmetically acceptable hydrophobic polymer thickener substantially free from cross-linking which is soluble in the solvent ; and

(iii) optionally, other ingredients in an amount not more than 50% by weight. Thus the solvent (i) and polymer thickener (ii) constitute from 50 to 100% by weight of the oleogel.

## DISCLOSURE OF THE INVENTION

The cosmetic oleogel according to the invention, in its simplest form comprises a blend of a special solvent chosen from selected natural fats and oils and/or modified fats and oils and/or other polyol fatty acid polyesters, together with a hydrophobic polymer thickener which is soluble in the solvent.

The oleogel so formed will generally retain its gel-like characteristics over a wide temperature range, for example from -15°C to + 60°C.

### The Solvent

The cosmetically-acceptable solvent is chosen, with certain exceptions, from polyol fatty acid polyesters, and mixtures thereof, the polyol from which the polyol fatty acid polyester is derived having at least 3 free hydroxyl groups, at least 60% of which are then esterified with one or more fatty acids having from 8 to 22 carbon atoms.

### Natural Oils and Fats

Those polyol fatty acid polyesters, as herein defined, which are derived from polyols having only 3 free hydroxyl groups are exemplified by animal, vegetable and marine oils and fats. This group of polyol fatty acid polyesters is however limited to those where at least one of the 3 free hydroxyl groups is esterified with a fatty acid chosen from alkanoic or alkenoic fatty acids having from 13 to 22 carbon atoms.

It is further stipulated that selected natural oils and fats that are suitable for use in the cosmetic oleogel according to the invention are chosen from those having an Iodine Value not exceeding 180 and preferably at least 5. Ideally the selected oil or fat will have an Iodine Value of from 10 to 150.

It is to be explained that when the oil is a vegetable oil or a marine oil, it will normally contain at least a proportion of unsaturated fatty acids or glycerides with an unsaturated fatty acid moiety, and therefore they will generally possess an Iodine Value which is generally >5. Very highly unsaturated oils having an Iodine Value of at least 180 or even higher, and linseed oil is an example, are not generally suitable for use in cosmetic products in view of their tendency to develop rancidity in a very short space of time. For this reason, oils having an Iodine Value in excess of 180 are excluded from the definition of the oils which can usefully be employed in the oleogel of the invention. Small amounts of such excluded oils can however be tolerated, provided that not more than 5% by weight of the solvent component of the oleogel, as herein defined, is constituted by such oils.

Examples of suitable animal fats, together with their respective Iodine Values (I.V), include:

| Animal Fats | I.V. |
|---|---|
| Beef tallow | 42 |
| Neat's foot oil | 72 |
| Pig lard | 55 |
| Mink oil | 66 |

Examples of suitable vegetable oils, together with their respective Iodine Values (I.V.), include:

| Vegetable Oil | I.V. |
|---|---|
| Rapeseed oil | 103 |
| Coconut oil | 9 |
| Cottonseed oil | 107 |
| Groundnut oil | 89 |
| Corn oil | 122 |
| Olive oil | 88 |
| Palm oil | 58 |
| Palm kernel oil | 17 |
| Sesame seed oil | 115 |
| Sunflower seed oil | 137 |
| Avocado pear oil | 94 |
| Neem oil | 68 |
| Safflower oil | 125 |
| Soya bean oil | 130 |
| Thistle seed oil | 133 |
| Cocobutter | 52 |
| Shea butter | 59 |
| Almond oil | 69 |
| Rice bran oil | 112 |

Examples of suitable marine oils, together with their respective Iodine Values (I.V) include:

| Marine Oil | I.V. |
|---|---|
| Monkfish liver oil | 162 |
| Coalfish liver oil | 167 |
| Codliver oil | 163 |
| Dogfish liver oil | 122 |
| Haddock liver oil | 168 |
| Halibut liver oil | 170 |
| Herring body oil | 154 |

It is understood that the above identified vegetable and marine oils are examples of suitable oils, most of which are readily available commercially, which can conveniently comprise the solvent in accordance with the invention, but that there exist many other examples of suitable oils which can be employed for this purpose.

Modified oils and fats

Those polyol fatty acid polyesters, as herein defined, which are derived from polyols having only 3 free hydroxyl groups are also exemplified by animal, vegetable and marine oils and fats that have been modified by industrial processing, for example by physical, chemical or enzymic treatment, to alter their physical and/or chemical properties, and generally to upgrade them to a form more suited for use in cosmetics or other topical products.

Examples of industrial processing include: transesterification, interesterification, full or partial hydrogenation, fractionation, deodorisation and decolouration (bleaching).

Specific examples of vegetable and marine oils that have been modified by industrial processing include:

deodorised, decolourised palm oil

hardened palm oil

partially hydrogenated fish oil

palmolein

Other polyol fatty acid polyesters

Other polyol fatty acid polyesters that can be employed as a solvent are those derived from any aliphatic or aromatic polyol which has at least 4 free hydroxyl groups, at least 60% of which are then esterified with one or more fatty acids having from 8 to 22 carbon atoms.

It is preferred that at least 60% of the free hydroxyl groups are esterified, as this renders these polyol fatty acid polyester resistant to cleavage by enzymes, particularly lipase, and as such, are particularly suitable for use in olegogels intended for application to the skin surface where lipases can exist.

The polyol from which these other polyol fatty acid polyesters are derived are preferably chosen from sugar polyols, which comprise mono-, di- and polysaccharides.

Preferred examples of monosaccharide sugar polyols include:

Pentose sugar polyols such as D-ribose, D-arabinose, D-xylose, D-lyxose, D-ribulose and D-xylulose.

Hexose sugar polyols such as D-allose, D-altrose, D-glucose, D-mannose, D-gulose, D-idose, D-galactose, D-talose, D-fructose, D-sorbose and D-tagatose. Heptose sugar polyols, such as D-mannoheptulose and D-sedoheptulose.

Preferred examples of disaccharide polyols include:

disaccharides such as maltose, lactose, cellobiose, sucrose, trehalose, gentiobiose, melibiose and primeverose.

Preferred examples of polysaccharide polyols include:

tri-saccharides, such as gentianose and raffinose.

The polyol from which the polyol fatty acid polyesters are derived can alternatively be chosen from:

sugar alcohols such as D-mannitol, D-sorbitol, D-ribitol, D-erithritol, D-lactitol and D-xylitol; and

derivatives of sugars such as -methyl glucoside and inositol.

The fatty acids which are employed to form the polyol fatty acid polyesters can be individual free fatty acids having from 8 to 22 carbon atoms in the fatty acid molecule.

These fatty acids can be saturated or unsaturated, linear or branched chain fatty acids.

A preferred source of fatty acids for forming these other polyol fatty acid polyesters are naturally occurring oils and fats which provide a source of a blend of fatty acids residues, whose choice can vary widely the physical and chemical properties of the polyol fatty acid polyesters obtained therefrom.

These oils and fats can be obtained from natural sources and used as such, or following chemical or enzymic treatment to provide modified oils and fats by full or partial hydrogenation, interesterification, transesterification or fractionation.

Suitable natural sources of these fatty acid residues may be of animal, vegetable or marine origin, such as tallow, lanolin oil, cod liver oil, halibut liver oil, other fish oils, coconut oil, palmkernel oil, palm oil, butter fat, soyabean oil, safflower oil, cotton seed oil, rapeseed oil, poppy seed oil, corn oil, sunflower oil, ground nut oil, fish liver oils and mixtures thereof. Preferred fatty acid sources are palm oils, partially hydrogenated palm oils, palm kernel oils, optionally partially hydrogenated soya bean oils and partially hydrogenated fish oils.

By employing a mixture of fatty acids, or one or more naturally occurring oils such as those exemplified above, when synthesising these other polyol fatty acid polyesters, it is possible to provide polyol fatty acid polyesters in which a mixture of ester groups is present on a single polyol molecule.

The polyol which can be reacted with a source of fatty acids such as those herein described will, as has previously been stated, comprise at least 4 free hydroxy groups, any or all of which are available for esterification with the fatty acid moieties. Usually, at least 60% of these free hydroxy groups are esterified to provide the polyol fatty acid polyester which is to be employed in forming the oleogel of the invention. More preferably, 70% and ideally at least 80% of these free hydroxy groups are substituted with fatty acid ester groups.

Preferred examples of these other polyol fatty acid polyesters include:

Sucrose octaisostearate

Sucrose octa-2-ethylhexanoate

Sucrose fatty acid polyesters derived from

palm and palmkernel oil mixtures

soyabean oil

soyabean and palm oil mixtures

palm oil

coconut oil, and

mixed fish oils

The amount of the solvent present in the oleogel in accordance with the invention is generally from 5 to 95%, preferably from 10 to 80% by weight.

## The Hydrophobic Polymer Thickener

The hydrophobic polymer thickener should be cosmetically acceptable and is preferably chosen from polymers which are substantially free from cross-linking and which are soluble in the solvent, as herein defined.

Examples of suitable polymer thickeners are:

polyethylene homopolymer, such as A-C Polyethylene 1702 (molecular weight 1700), A-C Polyethylene 617 (molecular weight 4300) and A-C Polyethylene 6 (molecular weight 5500), available from Allied Signal, and

polyethylene vinyl acetate copolymers, such as A-C Ethylene-vinyl acetate 405 (molecular weight 6500, co-monomer 6-11%), and A-C Ethylene-vinyl acetate 400 (molecular weight 6500, co-monomer 13%), also available from Allied Signal.

These polymer thickeners are preferably employed as a granular solid, which is suitable for addition to and solution in the solvent.

The amount of hydrophobic polymer thickener present in the oleogel in accordance with the invention is generally from 5 to 80%, preferably from 10 to 70% by weight.

## OTHER INGREDIENTS

The oleogel according to the invention can optionally comprise other ingredients to provide additional skin or hair benefit properties.

The oleogel according to the invention can also include healing agents, humectants, thickeners, antioxidants, stabilisers, film formers, emulsifiers, surfactants, sunscreens, preservatives, perfumes and colourants.

The oleogel according to the invention can also comprise other ingredients conventionally used in cosmetic products which are suited to topical application to human skin or hair.

Other ingredients, when present, can form up to 50% by weight of the composition and can conveniently form the balance of the ointment base.

## Process for preparing the ointment base

The invention also provides a process for the preparation of an oleogel suitable for topical application to skin or hair, which comprises the step of blending a polymer, as herein defined, with a solvent chosen from vegetable or marine oil having an Iodine Value of <180 and/or one or more other polyol fatty acid polyesters as herein defined.

According to a preferred embodiment of the process of the invention, the polymer and solvent are heated together with stirring to dissolve the polymer, and then cooled with continued stirring through the cloud point temperature of the mixture to obtain a transluscent oleogel. When the polymer is polyethylene, it is preferred to cool rapidly to obtain the oleogel having the best texture and appearance equivalent to that of petrolatum.

## Use of the oleogel

The oleogel according to the invention is intended primarily as a product for topical application to human skin, in particular to form an occlusive layer thereon, to reduce moisture loss. The skin can thereby be protected from adverse climatic conditions, for example from excessive exposure to sun and wind, or from detergent damage, for example that following immersion of the hands in aqueous detergent solution when washing dishes or clothes. A particular use for the oleogel of the invention is in the manufacture of colour cosmetics, lipsticks and lip salves.

The oleogel can also act as a carrier for sunscreen agents, perfumes or germicides or other skin benefit agents, whose presence on the skin surface can be prolonged due to the presence of the occlusive film, particularly when the solvent employed comprises one or more polyol fatty acid polyesters.

The oleogel can also be used to treat the hair and the scalp, particularly as a hair hold preparation or grooming aid, to maintain hair in a desired configuration or style.

In use, a small quantity of the oleogel, for example from 1 to 5g, is applied to the skin or hair from a suitable container or applicator and, if necessary, it is then spread over and/or rubbed into the skin or hair using the hand or fingers or a suitable spreading device.

## Product form and packaging

The oleogel of the invention can be formulated as a soft solid or jelly-like product having the rheological and other physical properties as herein defined, and it can be packaged in a suitable container to suit its viscosity and intended use by the consumer. For example, the oleogel can be stored in a deformable tube or in a lidded jar.

The invention accordingly also provides a closed container containing the oleogel as herein defined.

## Physical properties of the oleogels

### i) Consistency

The oleogel according to the invention should preferably have a Consistency Value of from 10 to 100, as measured by the Consistency Value Test. Oleogels having a Consistency Value of less than 10 are generally considered to be too hard for general cosmetic use, while oleogels having a Consistency Value of greater than 100 are generally too soft for use where a firm consistency is required.

The method used for measuring the Consistency Value of the oleogel, which has generally the same rheological properties and consistency of petrolatum, is described below.

### Consistency Value Test

The Consistency Value of the oleogel is measured by a cone penetrometer, whose construction and use will now be described.

The consistency of the oleogel of the invention is measured by means of a penetrometer, such as a Model PNR6, supplied by Sommer & Runge KG., Berlin fitted with a polished cone-shaped metal plunger weighing 116g, having a detachable steel tip with an internal angle of 120°C. The containers for the test are flat-bottomed metal or glass cylinders that are 102 ± 6mm in diameter and not less than 60mm in height.

The procedure for carrying out the Consistency Value Test is as follows:

Melt a quantity of the oleogel to be tested at a temperature of 82 ± 2.5°C, pour into one or more containers, filling to within 6mm of the rim. Cool to 25 ± 2.5°C over a period of not less than 16 hours, protected from draughts. Two hours before the test, place the containers in a water bath at 25 ± 0.5°C. If the room temperature is below 23.5°C or above 26.5°C, adjust the temperature of the cone to 25 ± 10.5°C by placing it in the water bath.

Without disturbing the surface of the oleogel under test, place the container on the penetrometer table, and lower the cone until the tip just touches the top surface of the test oleogel at a spot 25mm to 38mm from the edge of the container. Adjust the zero setting and quickly release the plunger, then hold it free for 10 seconds. Secure the plunger and read the total penetration from the scale. Make three or more trials, each so spaced that there is no overlapping of the areas of penetration. When the penetration exceeds 20mm, use a separate container of the test composition for each trial. Read the penetration to the nearest 0.1mm. Calculate the average of the three or more readings, and conduct further trials to a total of ten if the individual results differ from the average by more than ±3%: the final average of the trials is not less than 1.0mm and not more than 10.0mm, indicating a Consistency Value of from 10 to 100.

ii) Occlusivity

The oleogel according to the invention should normally have a significant Occlusivity Value, if it is intended that they are to be employed, like petrolatum, to provide an occlusive layer on skin or elsewhere on the body surface. Usually, the oleogel will have an Occlusivity Value of at least 30%, as measured by the Occlusivity Value Test. Preferred oleogels possess an Occlusivity Value of at least 50%, most preferably at least 60% and ideally of at least 70% as measured by this Test. Details of how this test is performed are given below.

Occlusivity Value Test

In view of the wide variation in the characteristics and properties of human skin, as seen amongst a group of individuals of differing ages, races and habitat, it is necessary to provide a standard in vitro test which is readily reproducable, in order to measure the occlusivity of the oleogel.

An empirical test has accordingly been devised using a standard viscose cellulose film, namely Visking dialysis tubing available from Medicell International Ltd. as a substitute for human skin. This film has a molecular weight cut-off of from 12,000 to 14,000.

In this test, the occlusivity of a film of the oleogel to the passage of water vapour applied to the dialysis film is measured in a standard manner as follows:

Preparation of occlusivity cell

A 5ml beaker, for example a Dispo beaker available from American Scientific Products, the diameter of the open end of which is 25mm (i.e. an area of ~5cm$^2$), is used to provide an occlusivity cell.

1ml distilled water is introduced into the beaker and a film of Visking dialysis tubing is stretched across the open end of the beaker and fixed in place with adhesive, for example Assembly Aid Adhesive (3M).

The rate of water loss through the Visking film at 20°C, at atmospheric pressure and at 50% external relative humidity, is determined by measuring the decrease in weight of the beaker with time using a Sartorius 4503 microbalance, with a D to A converter feeding the output to a chart recorder.

After a steady-state water loss rate has been established, a product whose Occlusivity Value is to be tested, i.e. the oleogel of the invention is applied as a film to the surface of the Visking dialysis tubing. When the test substance is liquid or a soft solid, it can be applied using a plastic-gloved finger. When the test material is a solid, it is necessary first to melt it as it is applied to the surface of the Visking dialysis film.

The new steady-state water loss rate, under the same physical conditions of pressure, temperature and relative humidity, is then recorded after excess water from the product has been lost.

Occlusivity of the product film (i.e. the oleogel) is then calculated as:

$$\% \text{ occlusivity} = 1 - \left[ \frac{\text{water loss rate with product}}{\text{water loss rate without product}} \right] \times 100$$

All water loss rates are corrected for the relatively small rate of water loss through the walls of the beaker (if any). This is determined by measuring the water loss from a beaker where the Visking film is replaced with impermeable aluminium foil.

Occlusivity is normally determined 4 times for each sample. For each measurement, the sample loading is determined from the increase in recorded weight immediately after application to the Visking film of the composition of the invention. Since the loading is not reproducable precisely, a straight line is fitted to a loading versus occlusivity plot (by linear regression) and the occlusivity at a typical consumer product loading of 10g/sq m is then interpolated. In each case, the occlusivity is approximately linearly dependent on the loading for the range covered.

The occlusivity is then expressed as an arithmetic mean of the 4 determinations ± 2 standard errors for 95% significance.

Experience has shown that about 10mg of the product applied to the Visking film is sufficient to provide an occlusive layer; without an occlusive layer, the film will normally transmit about 25g water vapour/m$^2$/hr.

EXAMPLES

The invention is illustrated with reference to the following examples in accordance with the invention. In each case, a blend was prepared by mixing together a polymer and a polyol fatty acid polyester and/or a vegetable oil.

For each formulation, the Occlusivity Value and Consistency Value were each determined by the respective Test, as described herein.

Examples 1, 2 & 3

These examples illustrate oleogels in accordance with the invention prepared from an oil, or oils and sucrose polyester as solvent, thickened with polyethylene as the polymer thickener.

In each example, the oleogel was prepared as follows:

i) Heat the solvent and polyethylene until a clear, homogenous solution results.

ii) While stirring, cool as rapidly as possible through the "cloud point" (temperature at which a clear, hot solution begins to get hazy as a result of precipitation of the polymer). By this means, the smallest possible particle size of the polymer is obtained and this results in a more stable, "stiffer" and smoother gel, than would otherwise result from more gradual cooling.

Gels with increased stability are formed if stirring is discontinued at about 10°C below the cloud point.

Rapid cooling (also known as shock cooling), to obtain relatively stable gels is achieved by immersing the vessel containing the solvent-polymer mixture into an ice bath whilst stirring, or by placing the vessel in a deep freeze cabinet at about -10°C. Alternatively, for large scale production, rapid cooling can be achieved by passing small containers containing the solution of the polymer through a cooling tunnel.

The formulation of each of the examples 1, 2 and 3, together with their respective Occlusivity Values and Consistency Values are given below in Table 2.

TABLE 2

| | Example Formulation | % w/w | Occlusivity Value | Consistency Value |
|---|---|---|---|---|
| 1. | Polyethylene 1702* | 50 | | |
| | | | 68 | 12 |
| | Sunflower seed oil | 50 | | |
| 2. | Polyethylene 617* | 17.5 | | |
| | Sucrose polyester** | 54.5 | 54 | 19 |
| | Sunflower seed oil | 28.0 | | |
| 3. | Polyethylene 1702* | 33.3 | | |
| | Sucrose polyester *** | 33.3 | 66 | 56 |
| | Sunflower seed oil | 33.3 | | |

\*      available from Allied Signal Inc.

\*\*     derived from soya bean oil

\*\*\*    derived from palm oil/palm kernel oil

Examples 4 & 5

These examples illustrate oleogels in accordance with the invention prepared from an oil, or oils and sucrose polyester as solvent, thickened with polyehtylene/vinyl acetate copolymer as the polymer thickener.

In each example, the oleogel was prepared as follows:

i) Heat the solvent and polyethylene/vinyl acetate copolymer until a clear, homogenous solution results.

ii) At just above the "cloud point", scrape the inside of the vessel containing solvent and polymer whilst cooling the outside of the vessel with a stream of water at about 18°C or lower. This removes the layer of gel formed on the cool wall, thus allowing the still liquid interior of the solution then to come into contact with the cool wall to promote the gelling of a further layer of oleogel. This procedure is continued until the entire solution has been converted to an oleogel.

It was noted that the extreme or rapid cooling applied as described in Examples 1, 2 and 3 above is not necessary when preparing oleogels using the polyethylene/vinyl acetate copolymer as the polymer thickener.

The formulation of each of the examples 4 and 5, together with their respective Occlusivity Values and Consistency Values are given below in Table 3.

TABLE 3

| | Example Formulation | % w/w | Occlusivity Value | Consistency Value |
|---|---|---|---|---|
| 4. | Polyethylene vinyl acetate copolymer* | 25 | | |
| | | | 40 | 21 |
| | Sunflower seed oil | 75 | | |
| 5. | Polyethylene vinyl acetate copolymer* | 13 | | |
| | Sucrose polyester*** | 17.4 | 40 | 20 |
| | Sunflower seed oil | 69.6 | | |

\*   available from Allied Signal Inc.

\*\*\* derived from palm oil/palm kernel oil

The rheology of compositions according to these examples, was examined using a CARRIMED controlled stress rheometer which determined viscosity at a range of shear rates. It was found that the viscosity values closely resembled those for petrolatum.

The melting profiles were determined by NMR proton relaxation, and found to resemble that for petrolatum which displays only a slow diminution in the amount of crystalline material as its temperature rises. Another test of melting properties entailed heating a thermometer bulb coated with the composition until the coating was mobile enough to form a droplet which falls off from the thermometer bulb. In this test the compositions of Examples 4 and 5 did not form a droplet until reaching approximately 77°C whereas petrolatum formed a droplet at 64°C. Thus these examples of the invention displayed better temperature stability than petrolatum.

## Claims

1. A cosmetic oleogel suitable for topical application to the human body surface, which comprises:
   (i) from 5 to 95% by weight of a cosmetically acceptable solvent chosen from polyol fatty acid polyesters, and mixtures thereof, the polyol having at least 3 free hydroxyl groups, at least 60% of which are then esterified with one or more fatty acids having from 8 to 22 carbon atoms;
   provided that when the polyol fatty acid polyester is derived from a polyol having 3 free hydroxyl groups, then at least one fatty acid with which it is esterified is chosen from alkanoic or alkenoic fatty acids having from 13 to 22 carbon atoms; the polyol fatty acid polyester having an Iodine Value of <180;
   (ii) from 5 to 70% by weight of a cosmetically acceptable hydrophobic polymer thickener substantially free from cross-linking which is soluble in the solvent; and
   (iii) optionally, from 0 to 50% by weight of other ingredients.

2. An oleogel according to claim 1, in which the solvent is chosen from polyol fatty acid polyesters derived from a naturally occurring triglycerides.

3. An oleogel according to claim 2, in which the triglyceride is chosen from animal, vegetable and marine oils and fats.

4. An oleogel according to claim 3, in which the animal oils and fats are chosen from:

Beef tallow
Neat's foot oil
Pig lard
Mink oil

5. An oleogel according to claim 3, in which the vegetable oil is chosen from:
Rapeseed oil
Coconut oil
Cottonseed oil
Groundnut oil
Corn oil
Olive oil
Palm oil
Palm kernel oil
Sesame seed oil
Sunflower seed oil
Avocado pear oil
Neem oil
Safflower oil
Soya bean oil
Thistle seed oil
Cocobutter
Shea butter
Almond oil
Rice bran oil

6. An oleogel according to claim 3, in which the marine oil is chosen from:
Monkfish liver oil
Coalfish liver oil
Codliver oil
Dogfish liver oil
Haddock liver oil
Halibut liver oil
Herring body oil

7. An oleogel according to any of claims 2 to 6, in which the triglyceride is modified by a process chosen from transesterification, interesterification, full or partial hydrogenation, fractionation, deodorisation and decouration (bleaching).

8. An oleogel according to claim 1, in which the solvent is chosen from polyol fatty acid polyesters, the polyol having at least 4 free hydroxyl groups, at least 60% of which are then esterified with one or more fatty acids having from 8 to 22 carbon atoms.

9. An oleogel according to claim 8, in which the polyol fatty acid polyester is chosen from:
Sucrose octaisostearate
Sucrose octa-2-ethylhexanoate
Sucrose fatty acid polyesters derived from
palm and palmkernel oil mixtures
soyabean oil
soyabean and palm oil mixtures
palm oil
coconut oil, and
mixed fish oils

10. An oleogel according to claim 1, in which the polymer thickener is a polyethylene.

11. An oleogel according to claim 1, in which the polymer thickener is a polyethylene vinyl acetate copolymer.

12. The use of an oleogel according to any preceding claim, for providing an occlusive layer on human skin, hair or nails following topical application thereto of the oleogel.

13. The use of an oleogel according to any of claims 1 to 12, as a vehicle for a protection agent or a benefit agent for topical application for human skin, hair or nails.

**European Patent Office**

## EUROPEAN SEARCH REPORT

Application Number

EP 92 30 5625

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 458 600 (UNILEVER PLC.) <br> * the whole document * <br> --- | 1-13 | A61K7/48 <br> A61K7/06 <br> A61K7/043 |
| X | EP-A-0 265 702 (KAO CORPORATION) <br><br> * the whole document * <br> --- | 1-3,10, 12,13 | |
| X | EP-A-0 391 431 (KAO CORPORATION) <br><br> * the whole document * <br> --- | 1-3,10, 12,13 | |
| X | GB-A-2 185 683 (REDKEN LABORATORIES INC.) <br><br> * the whole document * <br> --- | 1,2,10, 12,13 | |
| X | EP-A-0 277 335 (KAO CORPORATION) <br><br> * the whole document * <br> --- | 1-3,7, 10,12,13 | |
| D,X | GB-A-1 370 699 (DYNAMIT NOBEL AKTIENGESCHELLSCHAFT) <br> * the whole document * <br> --- | 1-3,10, 12,13 | TECHNICAL FIELDS SEARCHED (Int. Cl.5 ) |
| P,A | EP-A-0 466 410 (UNILEVER PLC.) <br><br> * the whole document * <br><br> ----- | 1-3,8,9, 12,13 | A61K <br> C07H |

The present search report has been drawn up for all claims

| Place of search | Date of completion of the search | Examiner |
|---|---|---|
| THE HAGUE | 05 OCTOBER 1992 | COUCKUYT P.J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)